# EUROPEAN PATENT APPLICATION

(11) **EP 4 491 198 A1**
(43) Date of publication of application: **15.01.2025**
(21) Application number: 23185240.1
(22) Date of filing: 13.07.2023
(51) Int. Cl.: A61K 47/58, A61K 9/14, A61K 31/10, A61K 47/60, A61P 39/04, C07D 341/00

(54) **METAL SCAVENGER**

(71) Applicant: PharmaLundensis AB, 223 63 Lund (SE)
(72) Inventor: SKOGVALL, Staffan, 226 48 LUND (SE); AXELSSON, Oskar, 243 32 HÖÖR (SE)
(74) Representative: Ström & Gulliksson AB

(57) **Abstract**

The present disclosure relates to a scavenger comprising a compound according to Formula I coupled to a carrier resin, wherein m, n, o and p independently from each other are 2 or 3; q is 1 or 2; X is -S- or >N(~); and A is >CH(CH₂O∼) when X is -S-, and A is -CH₂- when X is >N(~); wherein ~ represents a bond, optionally via a linker, to the carrier resin; and > denotes bonds within Formula I; and wherein the carrier resin is a polymer comprising hydrophilic groups or is a resin having a total molecular composition of (a+b)/(c) < 0.32, where a, b, and c are the mol percentage of oxygen (O), the mol percentage of nitrogen (N), and the mol percentage of the sum of all non-hydrogen atoms, respectively. The present disclosure also relates to scavengers for use as a medicament and to a method of preparation of a compound according to Formula I.

## Description

### Technical field

The present disclosure relates to scavengers that bind heavy metals, especially mercury, and to methods of producing such scavengers. The present disclosure also relates to the use of such scavengers in scavenging heavy metals and to the use of such scavengers as medicaments.

### Background

Mercury is an element that occurs naturally in the earth's crust. As a consequence, large amounts of elemental mercury vapours are emitted to the air during volcanic eruptions. Mining and fossil fuel combustion, as well as many other industrial activities, also release significant amounts of mercury into the atmosphere. Mercury emitted into the air eventually settles into water or onto land where it can be washed into water. Once deposited, certain microorganisms can change the elemental mercury to methylmercury, a highly toxic form that builds up in sea food such as fish and shellfish, as well as in animals, and in humans who eat them.

Almost all humans in the world have at least trace amounts of methylmercury in their tissues. Methylmercury is considered to cause neurological diseases such as learning disabilities in children. It has also been suspected to cause many other neurological and non-neurological diseases in humans.

Mercury has a long *in vivo* half-life because it can transform into a multitude of species like Hg²⁺, MeHg⁺, Me₂Hg, and MeHg-cysteine where some are highly lipophilic and will stay for a long time in various lipid-rich compartments of the body. Methylmercury is readily absorbed into the body after ingestion or inhalation. It has a long half-life of up to 80 days in humans. It is mainly excreted in the bile coupled to glutathione. However, after excretion in the bile, the methylmercury-glutathione complex is to a significant degree reabsorbed from the intestine, thereby creating an entero-hepatic recirculation. This considerably impairs the ability to excrete methylmercury and prolongs the excretion time.

Resins containing thiourea and thiouronium groups are used to bind mercury in wastewater. Thiourea/isothiouronium is a well-known carcinogen. Resin-bound thiourea and isothiouronium salts are expected to shed thiourea/isothiouronium *in vivo* due to enzymatic degradation in the intestinal tract. Thus, these substances are not suitable for medical use since they are unstable in the body and can be expected to degrade in contact with the intestinal fluid.

Iodinated activated charcoal can be used to remove mercury from polluted smoke in industrial processes. However, this substance is unstable in the human body, releasing large amounts of iodine which affects the thyroid function negatively. Thus, this substance is unsuitable for medical use.

Various thiolated resins are available and although they can be predicted to bind mercury, they will also bind essential metal ions such as Zn²⁺ and Cu²⁺, which makes them less suitable for long-term medical treatment.

Small-molecule chelators, such as dimercaprol, also called British anti-Lewisite (BAL), dimercaptosuccinate (DMSA), dimercaptopropane sulfonate (DMPS) and D-penicillamine are currently used to remove bulk mercury from the organism in an acute phase of a mercury poisoning. However, the use of these molecules for treating mercury poisoning has several drawbacks. BAL requires deep intra-muscular injection that is extremely painful and allergenic. A major drawback associated with DMSA is its extracellular distribution, since it is unable to cross the cell membrane. Adverse reaction of DMSA include gastrointestinal discomfort, skin reaction, mild neutropenia and elevated liver enzymes. Furthermore, the treatment with DMSA after exposure to inorganic mercury causes an increased elevation of mercury into motor axons. Adverse reactions during treatment with DMPS include gastrointestinal discomfort, skin reactions, mild neutropenia, and elevated liver enzymes. Some patients, especially those with allergic asthma symptoms, may develop hypersensitivity to DMPS. Furthermore, DMPS is suspected to be a human mutagen which prevents chronic treatment. D-penicillamine has been reported to cause anorexia, nausea and vomiting as well as more serious side-effects such as thrombocytopenia and leukocytopenia and rarely aplastic anemia.

Thus, there is a need to provide means to increase the excretion of methylmercury from the body and to remove mercury from an organism using a less toxic treatment or even non-toxic treatment.

### Summary

According to a first aspect, the above and other objects of the invention are achieved, in full or at least in part by a composition as defined by claim 1. According to this claim, the above object is achieved by a scavenger comprising a compound according to Formula I coupled to a carrier resin, 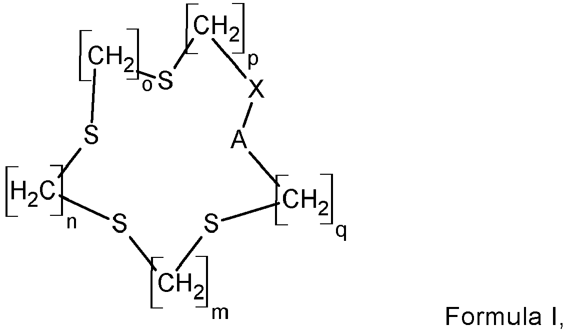 wherein m, n, o and p independently from each other are 2 or 3; q is 1 or 2; X is -S- or >N(~); and A is >CH(CH₂O∼) when X is -S-, and A is - CH₂- when X is >N(~); wherein ~ represents a bond, optionally via a linker, to the carrier resin; and > denotes bonds within Formula I; wherein the carrier resin is a polymer comprising hydrophilic groups.

Such scavengers have been shown to chelate heavy metals, particularly mercury and may thus be used to remove or reduce the concentration of heavy metals in different materials. The heavy metal may be chosen from the group comprising mercury (Hg), cadmium (Cd), arsenic (As), chromium (Cr), thallium (Tl), and lead (Pb).

The presence of hydrophilic groups in the carrier resin increases the ability to scavenge the heavy metal.

According to one embodiment, the hydrophilic groups have a molecular composition of (a+b)/c ≥ 0.25 where a, b, and c are the mol percentage of oxygen (O), the mol percentage of nitrogen (N), and the mol percentage of the sum of all non-hydrogen atoms, respectively.

According to one embodiment, the hydrophilic groups are polyethylene glycol (PEG), preferably wherein the polyethylene glycol has a polymerization degree of 5-100.

The carrier may be chosen from the group consisting of cross-linked hydrophilic polymers lacking hydrolysable bonds, such as PEG-polystyrene, crosslinked polyvinyl alcohol, or acrylates such as crosslinked polyacrylic acid.

According to one embodiment, the carrier resin comprises polystyrene. Such scavengers have been demonstrated to be particularly effective in scavenging heavy metals such as mercury.

The carrier resin may be in the form of beads, particles, or powder, preferably having a size of 20 µm to 2 mm.

According to on embodiment, m+n+o+p=10 or wherein m+n+o+p=8. In other words, two of m, n, o and p are 2 and the other two are 3 or all of m, n, o and p are 2. Preferably, m+n+o+p=8.

According to another embodiment, X is -S-.

According to another embodiment, m and p are 3; n and o are 2; q is 1; and X is -S-; or m and o are 3; n and p are 2; q is 1; and X is >N(~).

According to a further embodiment, m, n, o and p are 2; and q is 1. Preferably, X is -S-.

In a second aspect of the present disclosure, a scavenger for use as a medicament is provided. The scavenger comprises a compound according to Formula I coupled to a carrier resin, 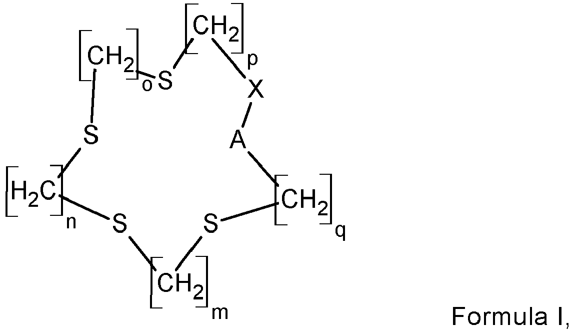 wherein m, n, o and p independently from each other are 2 or 3; q is 1 or 2; X is -S- or >N(~); and A is >CH(CH₂O∼) when X is -S-, and A is - CH₂- when X is >N(~); wherein ~ represents a bond, optionally via a linker, to the carrier resin; and > denotes bonds within Formula I; wherein the carrier resin is a resin having a total molecular composition of (a+b)/(c) < 0.32, where a, b, and c are the mol percentage of oxygen (O), the mol percentage of nitrogen (N), and the mol percentage of the sum of all non-hydrogen atoms, respectively.

The scavenger for use as a medicament as disclosed herein relies on the entero-hepatic system to deliver heavy metals, such as mercury, via the bile to the intestines where the orally administered, non-bioavailable, resin-bound scavenger according to the present disclosure can bind the heavy metal, such as mercury.

By oral intake of such a scavenger, heavy metals, present in the gastrointestinal tract may be chelated by the scavenger and removed from the body through the faeces. Thus, the scavengers may reduce the entero-hepatic recirculation of e.g. mercury species.

Further, since such a scavenger can be expected to be inert and therefore harmless to the body, it can be administered repeatedly for a long period of time, gradually depleting the heavy metal depots in the body without the risk of toxic side-effects, such as mobilizing heavy metal species, such as mercury species, from slow deposits.

The scavenger according to the present disclosure has no oral bioavailability. Thus, the scavenger gives no systemic toxicity, in contrast to the small-molecule chelators currently used to treat heavy metal poisoning. Hence, the scavenger according to the present disclosure can be used for a long period of time to remove heavy metals, e.g. mercury, from slowly eliminating compartments in the body.

Furthermore, the scavenger according to the present disclosure is stable under the conditions of the intestinal tract and will not disintegrate to any significant degree into potentially harmful compounds.

The daily dose may be from 0.01 to 100 g, such as from 0.03 to 50 g, such as 0.05 to 25 g. such as from 0.1 to 10 g of the scavenger per day, optionally broken up to more than one dosing per day. Other possible dosing schedules is once every few days such as every two, three, or seven days. The dosing may also be done as cycles with dosing from a week to a few months followed by a break during which the status of the status patient is evaluated and the need for one or more cycles is determined.

Such a scavenger may also be used in the extracorporeal removal of heavy metal, such as mercury. The scavenger is placed in a container, and blood from a subject is thereafter passed through the container. The blood may be passed through the container by hemoperfusion. When the blood comes into contact with the scavenger, heavy metal, such as mercury, is chelated by the scavenger and thus removed from the blood. The blood is thereafter returned to the blood circulation of the subject. In this way, the concentration of the heavy metal in the blood of the subject is reduced.

The carrier resin may be a resin having a total molecular composition of (a+b)/(c) ≥ 0.25, but < 0.32, where a, b, and c are the mol percentage of oxygen (O), the mol percentage of nitrogen (N), and the mol percentage of the sum of all non-hydrogen atoms, respectively.

According to one embodiment, the carrier resin is chosen from the group consisting of polystyrene or PEG-polystyrene.

Preferably, the carrier resin is PEG-polystyrene.

According to one embodiment, m+n+o+p=10 or wherein m+n+o+p=8. In other words, two of m, n, o and p are 2 and the other two are 3 or all of m, n, o and p are 2. Preferably, m+n+o+p=8.

According to one embodiment, X is -S-.

According to another embodiment, m, n, o and p are 2; q is 1. Preferably, X is -S-.

According to a further embodiment, m and p are 3; n and o are 2; q is 1; and X is -S-; or m and o are 3; n and p are 2; q is 1; and X is >N(~).

According to a further embodiment, the scavenger is comprised in a formulation chosen from the group consisting of a capsule, a tablet, a powder, a suspension, and an effervescent tablet.

According to a further embodiment, the scavenger is for use in the treatment and/or alleviation and/or prevention of diseases and/or conditions chosen from the group consisting of autoimmune diseases, neurological diseases, fibromyalgia, chronic fatigue syndrome, chronic obstructive pulmonary disease, chronic bronchitis, and heavy metal poisoning. The heavy metal may be chosen from the group comprising mercury (Hg), cadmium (Cd), arsenic (As), chromium (Cr), thallium (Tl), and lead (Pb).

According to another embodiment, the scavenger is for use in the treatment and/or alleviation and/or prevention of heavy metal poisoning, wherein the heavy metal is mercury.

In a third aspect of the present disclosure there is provided a method of preparation of a compound according to Formula I, 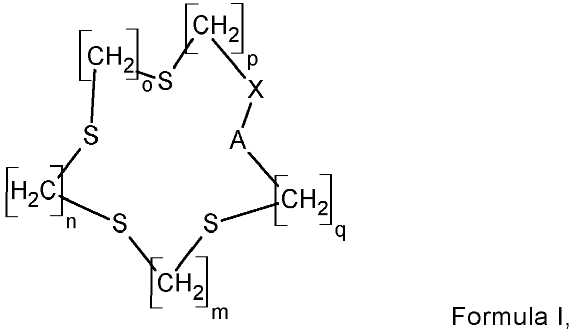
wherein m, n, o and p independently from each other are 2 or 3; q is 1 or 2; X is -S-; and A is >CH(CH₂O∼); wherein ~ represents H; and > denotes bonds within Formula I. The method comprises steps of (a) providing a solution of 1,2-dimercaptopropan-3-ol and a compound according to Formula II,

   MsO-(CH₂)ᵣ-S-(CH₂)ₛ-S-(CH₂)ₜ-S-(CH₂)ᵤ-OMs Formula II
wherein r, s, t, and u independently of each other are 2 or 3, in a polar aprotic solvent; (b) providing a solution of a non-nucleophilic base in a polar aprotic solvent; (c) adding the solution of step (a) to the solution of step (b) over a time period of 24 h or longer, while heating the mixture to a temperature of 50-150 °C; (d) removing the solvents; and (e) purifying the compound according to Formula I.

In step (a), 1,2-dimercaptopropan-3-ol and the compound according to Formula II may be provided in essentially equimolar amounts.

The polar aprotic solvent in step (a) may be DMF.

The non-nucleophilic base in step (b) may be chosen from the group consisting of DBU, DBN, or tetramethyl guanidine.

The polar aprotic solvent in step (b) may be DMF.

In step (c), the solution of step (a) may be added to the solution of step (b) over a time period of 48 h or longer.

Other objectives, features and advantages of the present disclosure will appear from the following detailed description, from the experimental data, as well as from the attached claims. It is noted that the disclosure relates to all possible combinations of features.

Generally, all terms used in the claims are to be interpreted according to their ordinary meaning in the technical field, unless explicitly defined otherwise herein. All references to "a/an/the [component, means, step, etc.]" are to be interpreted openly as referring to at least one instance of said component, means, step, etc., unless explicitly stated otherwise. The steps of any method disclosed herein do not have to be performed in the exact order disclosed, unless explicitly stated.

As used herein, the term "comprising" and variations of that term are not intended to exclude other additives, components, integers or steps.

### Definitions

A "linker" is a chemical bond or a collection of internally covalently bound atoms uniting two molecular moieties.

A "monomer" is a molecule that may bind covalently to other molecules of the same kind, (and optionally, other kinds) to form a polymer, i.e. a macromolecule composed of several monomer residues.

The term "monomer residue" refers to the atoms derived from one monomer unit as incorporated into the larger polymer. Depending on how the monomers link up, all the atoms may be retained or some may be lost as the bonds to the polymer form.

A "crosslink" refers to a link between two different chains in a polymer or between two different polymer chains. It is usually formed by the reaction of multifunctional monomers (i.e. crosslinkers) added when forming the polymer. Crosslinks may also be introduced e.g. by radiation treatment, chemical means, or heat.

The term "crosslinked" refers to a structure formed after the formation of at least one crosslink.

The term "covalently attached", "covalently linked" and "covalently bound" as used herein are synonymous, and the meaning thereof is well known to the skilled person.

The term "(selected) independent from each other" as used herein means that each of the different constituents mentioned is selected from the group of alternatives following after the term independently or separately from the selection of the other mentioned constituents.

The term "bioinert" as used herein refers to a material that is biocompatible, i.e. harmless to mammals and mammalian cells and at the same time stable to degradation *in vivo,* in a human (such as less than 10% degraded) for periods of one week or more.

The terms "hydrocarbon" and "hydrocarbon chain" are used herein to denote an organic residue comprising hydrogen and carbon. The hydrocarbon may be fully saturated or it may comprise one or more unsaturations. The hydrocarbon in accordance with the present invention may contain any number of carbon atoms between 1 and 50, such as 5 to 30, such as 10 to 20.

Numerical ranges: Whenever it is used herein, unless otherwise stated, a numerical range such as "1 to 8" or "1-8" refer to each integer in the given range; e.g., "1 to 8 atoms" and "1-8 atoms" mean that the group may consist of 1 carbon atom, 2 carbon atoms, 3 carbon atoms, etc., up to and including 8 carbon atoms. There are, however some exceptions which are clear to the skilled persons. In particular, whenever a range is given herein for a molar ratio, for a diameter or size, for a period of time, for a concentration, or for a temperature, the range includes also all decimal numbers falling within the range, including the upper and lower limits.

As used herein, the term "gel" means a cross-linked polymer where the covalent network extends through the whole object. Network polymer is a synonym for gel.

Phenol (or phenolic compound) is a trivial name for hydroxybenzene or, in a broader sense, any compound containing a hydroxysubstituted aromatic ring.

As used herein, the term "carrier resin" is defined as an insoluble, cross-linked, polymeric organic material, functionalized to be coupled with a heavy-metal chelating ring structure. Although such materials may swell in solvents such as water-dominated fluids, they will not dissolve into a microscopically homogenous solution.

As used herein, the term "hydrophilic group" refers to a group such that the total molar composition of this group fulfils the criterion: (oxygen + nitrogen / total number of non-hydrogen atoms) is higher than or equal to 0.25.

As used herein, the term "hydrophilic polymer", refers to a polymer or polymer group carrying hydrophilic groups such that the total molar composition of the polymer or polymer group fulfils the criterion: (oxygen + nitrogen / total number of non-hydrogen atoms) is higher than or equal to 0.25.

DBU is an acronym for diazabicycloundecene (1,8-diazabicyclo-[5.4.0]undec-7-ene).

DCM is an acronym for dichloromethane.

DMF is an acronym for dimethylformamide.

EtOH is an acronym for ethanol.

OMs is an acronym for methylsufonato-oxy.

PEG is an acronym for poly ethylene glycol.

Scavenging: removal of undesired impurities from a reaction mixture.

The term "scavenge" is to be understood as the action of removing an unwanted species from an environment by binding to a scavenger.

A "scavenger" is used to describe a carrier resin coupled with a heavy metal binding entity, used to remove said heavy metal from a system.

### Brief description of the drawings

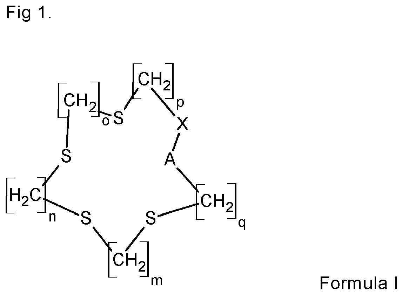

Fig. 1 shows a compound according to Formula I.

### Detailed description

The present disclosure relates to scavengers of heavy metal, in particular to biocompatible mercury scavengers, as well as the use of and methods of producing such scavengers.

Specific aspects and embodiments of the present disclosure will be described in detail below.

### Scavengers

The present disclosure relates to a scavenger comprising a compound according to Formula I coupled to a carrier resin, 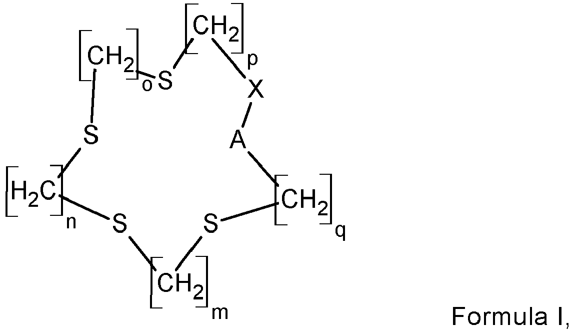 wherein m, n, o and p independently from each other are 2 or 3; q is 1 or 2; X is -S- or >N(~); and A is >CH(CH₂O∼) when X is -S-, and A is -CH₂-when X is >N(~); wherein ~ represents a bond, optionally via a linker, to the carrier resin; and > denotes bonds within Formula I.

The carrier resin is a polymer comprising hydrophilic groups or is a resin having a total molecular composition of (a+b)/(c) < 0.32, where a, b, and c are the mol percentage of oxygen (O), the mol percentage of nitrogen (N), and the mol percentage of the sum of all non-hydrogen atoms, respectively. Alternatively, the carrier resin is a resin having a total molecular composition of (a+b)/(c) ≥ 0.25, but < 0.32, where a, b, and c are the mol percentage of oxygen (O), the mol percentage of nitrogen (N), and the mol percentage of the sum of all non-hydrogen atoms, respectively. Alternatively, the resin is chosen from the group consisting of polystyrene or PEG-polystyrene.

When the carrier resin is a polymer comprising hydrophilic groups, the hydrophilic groups may be polyethylene glycol (PEG).

In certain embodiments, m+n+o+p=10 or m+n+o+p=8.

In other embodiments, q is 1.

In yet other embodiments, m, n, o and p are 2; and q is 1.

In specific embodiments, X is -S-.

In further embodiments, m, n, o and p are 2; q is 1; X is -S-, and A is >CH(CH₂O∼).

In yet other embodiments, m, n, o and p are 2; q is 1; X is >N(~), and A is -CH₂-.

In other embodiments, m and p are 3; n and o are 2; q is 1; X is -S-, and A is >CH(CH₂O∼).

In other embodiments, m and o are 3; n and p are 2; q is 1; X is >N(~), and A is -CH₂-.

In other embodiment m, n, o, p, q are all 2, X is -S-, and A is >CH(CH₂O∼).

In other embodiment m, n, o, p, q are all 2, X is -S-, A is >CH(CH₂O∼).

### Carrier resin

The carrier resin may be in the form of beads, particles, or powder, preferably having a size of 20 µm to 2 mm.

In specific embodiments, such when the scavenger is used for removal or reduction of heavy metal (further described below), the carrier resin is a polymer comprising hydrophilic groups. The hydrophilic groups may have a molecular composition of (a+b)/c ≥ 0.25 where a, b, and c are the mol percentage of oxygen (O), the mol percentage of nitrogen (N), and the mol percentage of the sum of all non-hydrogen atoms, respectively. Alternatively, the carrier resin is a polymer comprising hydrophilic groups resulting in a total molecular composition of (a+b)/(c) ≥ 0.25 where a, b, and c are the mol percentage of oxygen (O), the mol percentage of nitrogen (N), and the mol percentage of the sum of all non-hydrogen atoms, respectively. Examples of such resins include, but are not limited to, polyethylene glycol (PEG) ((a+b)/(c) = 1/3); a carrier resin comprising polystyrene comprising hydrophilic groups, e.g. PEG-polystyrene, such as PEGylated polystyrene with at least five PEG monomers/styrene monomer ((a+b)/(c) = 0.25). Further examples include polymethylmethacrylate ((a+b)/(c) = 1/3), polyvinyl alcohol: ((a+b)/(c) = 1/3), and polyvinylpyrrolidone ((a+b)/(c) = 0.25). Preferably, the hydrophilic groups of the carrier resin are polyethylene glycol (PEG). Preferably, the polyethylene glycol has a polymerization degree of 5-100. Specifically, the carrier resin may be a cross-linked hydrophilic polymer lacking hydrolysable bonds, e.g. PEG-polystyrene, crosslinked polyvinyl alcohol, or acrylate, such as crosslinked polyacrylic acid. Preferably, the carrier resin comprises polystyrene, such as PEG-polystyrene. Specific carrier resins include Tentagel^{®} and Hypogel^{®} and are described below.

In other specific embodiments, such as when the scavenger is used as a medicament (further described below), the carrier resin is a resin having a total molecular composition of (a+b)/(c) < 0.32, where a, b, and c are the mol percentage of oxygen (O), the mol percentage of nitrogen (N), and the mol percentage of the sum of all non-hydrogen atoms, respectively. The carrier resin may be a resin having a total molecular composition of (a+b)/(c) ≥ 0.25, but < 0.32, where a, b, and c are the mol percentage of oxygen (O), the mol percentage of nitrogen (N), and the mol percentage of the sum of all non-hydrogen atoms, respectively. Preferably, the carrier resin is chosen from the group consisting of polystyrene or PEG-polystyrene. In a preferred embodiment, the carrier resin is polystyrene-PEG. Specific carrier resins include Merrifield^{®}, Tentagel^{®} and Hypogel^{®} and are described below.

### Linker

The ring structures can be linked to the resin in many ways obvious to one skilled in the art. The prerequisite is that this link or linker is non-toxic and stable to the conditions encountered when passing through the human digestive tract. This excludes groups such as esters, amides, acetals, hemiacetals, peroxides, and disulfides, as critical parts of the linker.

Thus, preferably, the linker is a hydrolytically stable linker (i.e. stable to base such as 1 M aqueous NaOH or acids such as 1 M aqueous HCl, at 100 °C for one hour).

The linker may be attached to the polymer backbone, or to a side chain of the carrier resin such as to PEG in the case of PEG-Polystyrene.

Suitable linkers may comprise ether groups.

Other suitable linkers are hydrocarbons. It is desirable that the amount of hydrocarbon is not so large that the linker becomes hydrophobic.

Thus, the linker may comprise a hydrolytically stable chain of 1 to 40, such as 5 to 30, such as 10 to 25, such as 15 to 20, atoms chosen from C, O, N and S connected covalently and hydrogen added according to chemical valences. Preferably the ratio of (aO + bN + cS)/dC, wherein a, b, c and d denote the number of O, N, S and C atoms, respectively, is 0.2 or higher. Some examples of such linkers are -CH₂-O-, -CH₂-O-CH₂-, and -CH₂-SO₂-CH₂-.

It is also possible to include various stable ring structures in the linker. Such ring structures may be aromatic hydrocarbons; stable aromatic heterocycles like imidazole and pyridine; or aliphatic carbocyclic such as cyclopentyl and cyclohexyl; or stable aliphatic heterocycles such as morpholine, tetrahydropyran, and tetrahydrofuran. Such fragments can be linked up in any chemically reasonable order.

However, usually the linker is as simple as a covalent bond, a short sequence of CH₂-groups, such as 1 to 5, or one or more ether linkages.

In certain specific embodiments, the linker is a covalent bond.

### Specific scavengers

In a preferred embodiment, m, n, o, p, are all 2; q is 1; X is -S-; A is >CH(CH₂O∼); the linker is a covalent bond; and the carrier resin is polystyrene.

In a preferred embodiment, m, n, o, p, are all 2, q is 1, X is -S-, A is >CH(CH₂O∼), the linker is a covalent bond and the carrier resin is -PEG-polystyrene.

In a preferred embodiment, m, n, o, and p are all 2, q is 1, X is -S-, A is >CH(CH₂O∼), the linker is a covalent bond, and the carrier resin is - PEGs-polystyrene.

In a preferred embodiment, m, n, o, p, are all 2, q is 1, X is -S-, A is >CH(CH₂O∼), the linker is a covalent bond and the carrier resin is Merrifield^{®}, Tentagel^{®} or Hypogel^{®}. These carrier resins are as described below.

Further specific embodiments are described below.

### Removal or reduction of heavy metal concentration

A scavenger comprising a compound according to Formula I coupled to a carrier resin, 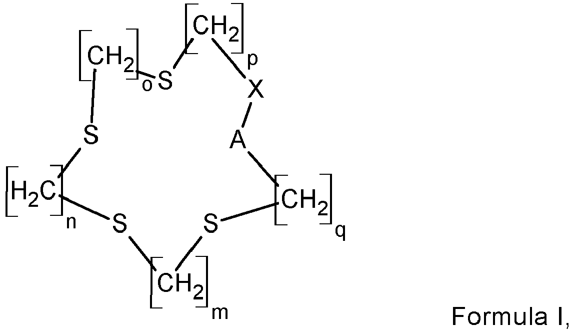 wherein m, n, o and p independently from each other are 2 or 3; q is 1 or 2; X is -S- or >N(~); and A is >CH(CH₂O∼) when X is -S-, and A is -CH₂-when X is >N(~); wherein ~ represents a bond, optionally via a linker, to the carrier resin; and > denotes bonds within Formula I; and wherein the carrier resin is a polymer comprising hydrophilic groups, may be used for removing or reducing the concentration of heavy metal from a gas, a liquid, a solution, a slurry, a gel, a powder or a material comprising particles.

The carrier resin may be as described above in relation to a polymer comprising hydrophilic groups.

The hydrophilic groups may be polyethylene glycol, preferably wherein the polyethylene glycol has a polymerization degree of 5-100.

Preferably, the carrier resin comprises polystyrene.

Suitable carrier resins are grafted copolymers consisting of a low crosslinked polystyrene matrix on which poly (ethylene glycol) (PEG) is grafted. Non-limiting examples include TentaGel^{®} and HypoGel^{®}, which are described below.

The linker may be as described above.

Specific ring structures of suitable scavengers are as described above.

The heavy metal may be chosen from the group comprising mercury (Hg), cadmium (Cd), arsenic (As), chromium (Cr), thallium (Tl), and lead (Pb).

Thus, the present disclosure also relates to a method of removing or reducing the concentration of heavy metal from a liquid, a solution, a slurry, a gel, a powder or a material comprising particles.

The gas, a liquid, a solution, a slurry, a gel, a powder or a material comprising particles is brought into contact with the scavenger according to the present disclosure and the heavy metal is chelated to the scavenger. When the gas, liquid, solution, slurry, gel, powder or material comprising particles is separated from the scavenger, the concentration of the heavy metal in the gas, liquid, solution, slurry, gel, powder or material comprising particles has been reduced.

The scavenger may be in the form of beads, particles, or powder, preferably having a size of 20 µm to 2 mm and is added to the liquid, the solution or the gel, preferably under mechanical agitation. After a certain period of time, which depends on the volume and on the amount of heavy metal to be scavenged, the beads, particles or powder are/is removed, often by being filtered off.

The scavenger may be present on a filter through which the liquid, the solution or the slurry is passed.

The scavenger may be present in a container through which the liquid, the solution or the slurry is passed.

A powder or a material comprising particles may be mixed with a solvent, such as water, and thereafter subjected to the scavenger according to the present disclosure as described above.

Specific examples of scavengers that can be used for removing or reducing the concentration of heavy metal from a gas, a liquid, a solution, a slurry, a gel, a powder or a material comprising particles are Scavengers **2-6** described below.

### Scavenger for use as a medicament

Heavy metals, such as mercury, is secreted from the body through different routes, such as through the faeces, urine or even by breathing.

Symptoms of mercury poisoning include muscle weakness, poor coordination, numbness in the hands and feet, skin rashes, anxiety, memory problems, trouble speaking, trouble hearing, or trouble seeing. Methylmercury exposure in children may result in acrodynia (pink disease) in which the skin becomes pink and peels. Long-term complications may include kidney problems and decreased intelligence. Mercury poisoning has also been suggested to be implicated in diseases such as autoimmune diseases, neurological diseases, fibromyalgia, chronic fatigue syndrome, chronic obstructive pulmonary disease, and chronic bronchitis.

Organic mercury, e.g. methylmercury, is mainly excreted in the bile coupled to glutathione. Inorganic mercury, such as mercury salts and metallic mercury, is excreted by other routes, such as from the blood via the kidneys to the urine. Mercury salts, such as such as mercuric chloride (HgCl₂) and mercurous chloride (Hg₂Cl₂), accumulate in the kidneys, and can cause severe kidney damage.

A scavenger comprising a compound according to Formula I coupled to a carrier resin, 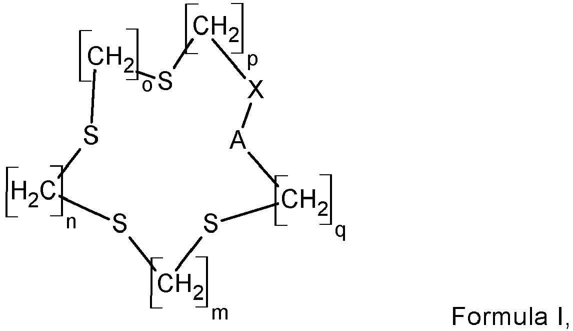 wherein m, n, o and p independently from each other are 2 or 3; q is 1 or 2; X is -S- or >N(∼); and A is >CH(CH₂O∼) when X is -S-, and A is -CH₂-when X is >N(∼); wherein ~ represents a bond, optionally via a linker, to the carrier resin; and > denotes bonds within Formula I; and wherein the carrier resin is a resin having a total molecular composition of (a+b)/(c) < 0.32, where a, b, and c are the mol percentage of oxygen (O), the mol percentage of nitrogen (N), and the mol percentage of the sum of all non-hydrogen atoms, respectively, may thus be used as medicament.

The carrier resin may be a resin having a total molecular composition of (a+b)/(c) ≥ 0.25, but < 0.32, where a, b, and c are the mol percentage of oxygen (O), the mol percentage of nitrogen (N), and the mol percentage of the sum of all non-hydrogen atoms, respectively.

Preferably, the resin is chosen from the group consisting of polystyrene or PEG-polystyrene.

The carrier resin may be as described above in relation to polystyrene or PEG-polystyrene.

Preferably, the carrier is polystyrene-PEG.

Non-limiting examples of suitable carrier resins include Merrifield^{®}, TentaGel^{®} and HypoGel^{®}, which are described below.

The linker may be as described above.

Specific ring structures of suitable scavengers are as described above.

The scavenger may be comprised in a formulation chosen from the group consisting of a capsule, a tablet, a powder, a suspension, and an effervescent tablet.

A scavenger according to the present disclosure is innocuous to the body and can thus be administered repeatedly for a long period of time, gradually depleting the heavy metal depots in the body without the risk of mobilizing heavy metal species, such as mercury species, from slow deposits.

The scavenger according to the present disclosure has no oral bioavailability. Thus, the scavenger gives no systemic toxicity. Hence, it can be used for a long period of time to remove heavy metals, e.g. mercury, from slowly eliminating compartments in the body.

Furthermore, the scavenger according to the present disclosure is stable under the conditions of the intestinal tract and will not disintegrate to any significant degree into potentially harmful compounds (as opposed to the small-molecule chelators currently used to treat heavy metal poisonings).

Such a scavenger may be used in the treatment and/or alleviation and/or prevention of diseases and/or conditions chosen from the group consisting of autoimmune diseases, neurological diseases, fibromyalgia, chronic fatigue syndrome, chronic obstructive lung disease, chronic bronchitis, and heavy metal poisoning.

Specifically, scavenger according to the present disclosure may be used in the treatment and/or alleviation and/or prevention of mercury poisoning.

Specific examples of scavengers that can be used as a medicament as described above are Scavengers **1-6** described below.

### Pharmaceutical formulations

The present disclosure also relates to pharmaceutical formulations comprising a scavenger comprising a compound according to Formula I coupled to a carrier resin, 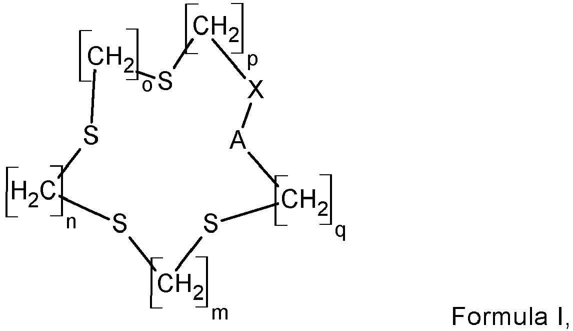 wherein m, n, o and p independently from each other are 2 or 3; q is 1 or 2; X is -S- or >N(∼); and A is >CH(CH₂O∼) when X is -S-, and A is -CH₂-when X is >N(∼); wherein ∼ represents a bond, optionally via a linker, to the carrier resin; and > denotes bonds within Formula I; and wherein the carrier resin is a resin having a total molecular composition of (a+b)/(c) < 0.32, where a, b, and c are the mol percentage of oxygen (O), the mol percentage of nitrogen (N), and the mol percentage of the sum of all non-hydrogen atoms, respectively,

The carrier resin may be a resin having a total molecular composition of (a+b)/(c) ≥ 0.25, but < 0.32, where a, b, and c are the mol percentage of oxygen (O), the mol percentage of nitrogen (N), and the mol percentage of the sum of all non-hydrogen atoms, respectively.

Preferably, the resin is chosen from the group consisting of polystyrene or PEG-polystyrene.

Preferably, the carrier is polystyrene-PEG.

The linker may be as described above.

The scavenger may be comprised in a formulation chosen from the group consisting of a capsule, a tablet, a powder, a suspension, and an effervescent tablet.

The pharmaceutical formulation may comprise flow-enhancers such as talcum, microcrystalline cellulose, starch, silica, or calcium phosphate. It may also comprise antioxidants such as ascorbic acid, polyphenols like gallic acid, sodium bisulfite, or sodium metabisulphite.

The pharmaceutical formulation may comprise disintegrants such as glucose, lactose, water soluble polymers or other suitable compounds that are hydrophilic enough to release the contents of a drug capsule or tablet.

The scavenger may be filled in capsules, preferably together with disintegrants such as glucose, lactose, water soluble polymers or other suitable compounds that are hydrophilic enough to release the contents of a drug capsule.

The scavenger may be pressed into tablets, preferably together with fillers, disintegrants, flow enhancers, lubricants and binders. Optionally, such tablets can be coated to facilitate swallowing.

The scavenger may be formulated as a suspension for oral administration.

The scavenger may be formulated for administration through an oral tube for the purpose of treating acute poisonings.

### Removal of heavy metal via the gastro intestinal tract

An effective method to increase the excretion of methylmercury from the body would be to reduce the reabsorption of the methylmercury-glutathione complex in the lower intestine, thereby breaking its entero-hepatic recirculation. In order to achieve this, a scavenger would need to have several properties. It should comprise an effective mercury-binding group. It should also comprise a carrier, such as a carrier resin, which is coupled to the mercury-binding group. This carrier should have properties that prevents it from being absorbed from the intestine into the blood, such as a high molecular weight. As a result, the scavenger will, after swallowing, simply be transported from the mouth through the stomach and intestine and be excreted in the faeces. The binding of mercury occurs in the upper part of the small intestine, where the scavenger gets in contact with the bile that contains the methylmercury-glutathione complex. Further, the scavenger should be bioinert, meaning that it is not toxic to the mucosa of the digestive tract, and is not broken down by the digestive enzymes of the body or by the intestinal bacteria. Finally, such a scavenger should bind mercury in aqueous solutions, since the intestinal fluid contains considerable amounts of water.

Scavengers according the present disclosure fulfil these criteria and are therefore suitable for removal of mercury from humans. The skilled person realizes that scavengers according to the present disclosure may also be suitable for removal of other heavy metals from humans.

By oral intake of a scavenger according to the present disclose, heavy metals such as mercury, present in the gastrointestinal tract may be chelated by the scavenger and removed from the body through the faeces.

Specifically, organic mercury, such as methylmercury, that has been excreted in the bile may be chelated by the scavenger and removed from the body through the faeces. Thereby the entero-hepatic recirculation of is interrupted and the total amount of methylmercury in the body is reduced.

The daily dose may be from 0.01 to 100 g, such as from 0.03 to 50 g, such as 0.05 to 25 g. such as from 0.1 to 10 g of the scavenger per day, optionally broken up to more than one dosing per day.

Typical dosing schedules for long-term dosing are from 0.1 to 10 g daily, optionally broken up to more than dosing per day. Other possible dosing schedules is once every few days such as every two, three, or seven days. The dosing may also be done as cycles with dosing from a week to a few months followed by a break during which the status of the status patient is evaluated and the need for one or more cycles is determined.

An advantage of a scavenger according to the current disclosure is its inert and non-toxic nature, which allows for treating acute heavy metal poisoning such as mercury poisoning with much higher doses in a hospital setting, where doses such as 50 g, or 100 g or even higher, may be administered by gavage at a single time and then optionally followed by more doses at the discretion of the treating physician.

In some embodiments the pharmaceutical formulation comprising a composition of formula 1 is administered as a daily dose of 0.01 to 100 g, such as from 0.03 to 50 g, such as 0.05 to 25 g. such as from 0.1 to 10 g for a period of 1-8 weeks.

In some embodiments the pharmaceutical formulation comprising a composition of formula 1 is administered as a daily dose of 0.1 to 1 g for a period of 1-8 weeks.

In some embodiments the pharmaceutical formulation comprising a composition of formula 1 is administered as a daily dose of 0.01 to 100 g, such as from 0.03 to 50 g, such as 0.05 to 25 g. such as from 0.1 to 10 g for a period of 1-8 weeks followed by a 1-8 weeks hiatus, whereupon this cycle is repeated, one or more times.

In some embodiments the pharmaceutical formulation comprising a composition of formula 1 is administered as a daily dose of 0.1 to 1 g for a period of 1-8 weeks followed by a 1-8 weeks hiatus, whereupon this cycle is repeated, one or more times.

### Extracorporeal removal of heavy metal

A more rapid and effective removal of heavy metal, such as mercury, from the body can be accomplished by extracorporeal binding of heavy metal. In such a case, a scavenger according to the present disclosure is placed in a container, and blood from a subject is thereafter passed through the container. According to one embodiment, the blood is passed through the container by hemoperfusion. When the blood comes into contact with the scavenger, heavy metal, such as mercury, is chelated by the scavenger and thus removed from the blood. The blood is thereafter returned to the blood circulation of the subject. In this way, the concentration of the heavy metal in the blood of the subject is reduced.

One advantage of extracorporeal removal of mercury is that mercury salts and metallic mercury may be removed from the circulation. Severe damage to e.g. the kidneys may thus be prevented or reduced.

The extracorporeal treatment may be carried out once to treat an acute exposure to a heavy metal.

The extracorporeal treatment may be carried out several times in order to remove large amounts of heavy metal, such as mercury, resulting from e.g. a chronic exposure to the heavy metal.

Thus, the present disclosure also relates to a device for removing a heavy metal, such as mercury, from the blood of a subject. The device comprises a container having an inlet and an outlet. The container encompasses a scavenger according to the present disclosure. Blood from the subject is passed into the container via the inlet and is brought into contact with the scavenger. Heavy metal compounds, such as mercury compounds, especially mercury salts and metallic mercury, are chelated by the scavenger. The blood leaves the container through the outlet and is thereafter returned to the subject.

The scavenger may be formulated for ex-vivo use where the body fluid of a patient is contacted with a bed of the scavengers according to the current disclosure, either in direct contact or in indirect contact via a dialysis membrane, and subsequently returned to the patient.

### Synthesis of ring structures

The ring structures according to Formula I 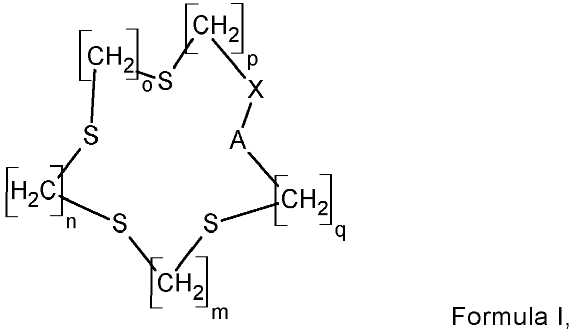 wherein m, n, o and p independently from each other are 2 or 3; q is 1 or 2; X is -S- or >N(H)-; and A is >CH(CH₂OH) when X is -S- and A is - CH₂- when X is >N(H), can be synthesized according to different strategies. Some non-limiting examples are outlined here.

### Ring structures of Formula 1, wherein A is >CH(CH₂OH) and X is -S-

The synthesis of ring structures of compounds according to Formula I, wherein A is >CH(CH₂OH) and X is -S-, which are used to prepare the scavengers **1-4,** involves the steps of:
1. Adding a mixture of 1,2-dimercaptopropan-3-ol and a compound according to Formula II

   MsO-(CH₂)ᵣ-S-(CH₂)ₛ-S-(CH₂)t-S-(CH₂)ᵤ-OMs Formula II,

   wherein r, s, t, and u each are 2 or 3,
   preferably in essentially equimolar amounts, dissolved in a polar aprotic solvent, such as DMF, to a solution of a non-nucleophilic base, such as DBU, DBN, or tetramethyl guanidine, in a polar aprotic solvent, such as DMF, at a slow rate, such as over 24 h, or 48 h or even longer, while heating the mixture to an elevated temperature such as 50-150 °C, such as 70-100 °C;
2. Removing the solvent; and
3. Purifying the reaction mixture to give a compound according to Formula I, wherein m, n, o and p independently from each other are 2 or 3; q is 1 or 2, and wherein A is >CH(CH₂OH) and X is - S-. The purification may be performed by established methods such as column chromatography, extraction, or crystallization.

This method has the advantage of producing a yield that is similar or higher than using the base caesium carbonate which is the standard in the field, while at the same time giving fewer side products which facilitates the purification.

This synthesis of the ring structures of compounds according to Formula I, wherein A is >CH(CH₂OH) and X is -S-, which are used to prepare the scavengers **1-4,** is exemplified for the compound wherein m, n o and p are 2 and q is 1 (used to prepare Scavenger **4**) and shown in Scheme 1. The dithiol is extended in a two-step sequence involving reaction of the precursor dithiol with a haloalcohol, such as bromopropanol or bromoethanol, followed by conversion to a leaving group, such as by mesylation. The critical ring-closures are performed by a slow addition of a mixture of the corresponding dimesylate and 1,2-dimercaptopropan-3-ol to a solution of a non-nucleophilic base, such as DBU. This is a considerable improvement over the published use of caesium carbonate, which leads to a very complex product mixture. The yield is similar but the purification of the resulting thiacrownether (in this case 2-hydroxymethyl-1,4,7,10,13-pentathiacyclopentadecane, see Example 4c below) is much more convenient. In order to produce the corresponding scavenger, the thiacrownether is coupled to a carrier. The couplings to the carriers is straightforward with generation of the anion of the ring structure and substitution of a halogen of the carrier precursor.

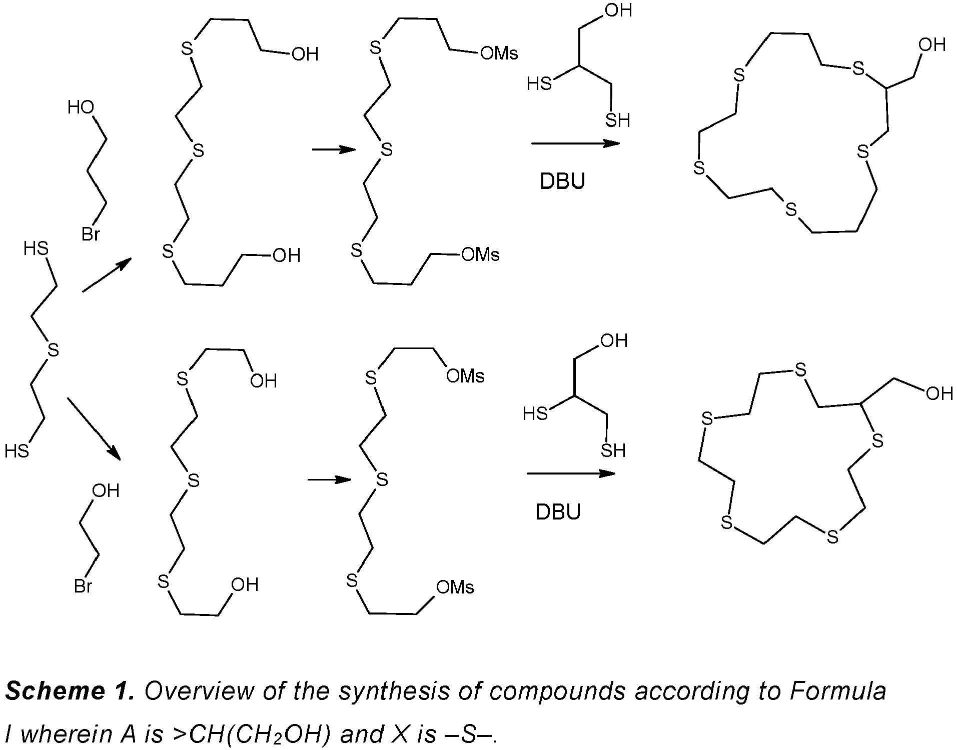

### Ring structures of Formula 1, wherein A is -CH2- when X is >N(H)

The syntheses of compounds according to Formula I, wherein A is -CH₂-when X is >N(H), and used to prepare the scavengers **5** and **6** are shown in scheme 2. An S₄ dithiol with two or three carbons, respectively, in the outer segments, are generated by standard methods. The critical ring-closures are performed by the slow addition of the dithiol and N-BOC-bis(2-chloroethyl)-amine to a solution of the non-nucleophilic base DBU. After deprotection, the coupling to the carriers are straight-forward, with the nucleophilic nitrogen substituting the halogen of the resin.

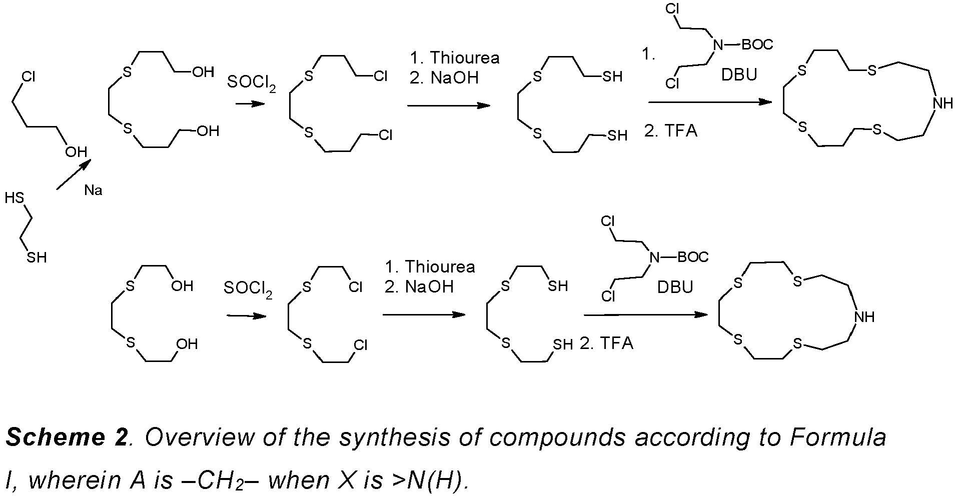

### Other synthetic routes

Other synthetic routes to the sulfur-ring systems of Schemes 1 and 2 may also be contemplated. The most practical would be to reverse the nucleophile-electrophile configuration of the cyclization step, having an S₅ fragment as the nucleophile and a double electrophile like epichlorohydrin, or a radical acceptor like propargylic alcohol, to close the ring.

### Preparation of scavengers - coupling to resin

The ring structures can be coupled to the resin via a covalent bond or via linker as described above.

To prepare the scavengers **1-6,** in which the ring structures are coupled to the resin via a covalent bond, the resin is suspended in DMF and, in another reaction flask, the ring structure and NaH in DMF at 0 °C are mixed and stirred at that temperature for at least 30 min. Thereafter, the mixture containing the ring structure is transferred to the resin and is left at rt for at least 24 hrs. The DMF is filtered off and the resin is optionally successively washed with diethyl ether, DCM and finally with water, and dried under vacuum to yield the scavenger.

For specific linkers, the reaction conditions and reagents are chosen depending on the linker used and are known to a person skilled in the art.

### Experimental data

### Measurement of mercury scavenging ability of scavengers 1-6.

Scavengers according to the present disclosure were prepared as described below and the ability to scavenger mercury from was assessed. Approximately 5 mg of the test compounds were weighed in in glass vials. The samples were incubated for 24 h at room temperature with 10 ml of a stock solution of HgCl₂ in PBS buffer. The incubation was terminated by separation of compound and solution by centrifugation. The concentrations of Hg in the solutions were measured with ICP-OES. A sample of the original stock solution of 15 µM HgCl₂ in PBS buffer, incubated in parallel in a glass vial, was used as a reference. The concentration of remaining Hg was defined as % recovery of the Hg. The tests were performed as triplicates and the results are reported in Tables 1 and 3.

The structures of the precursor resins are shown in Table 1 and the halogen atom is the reactive group that is substituted with the ring system and linker of Formula I. As can be seen in Table1, the tested resins scavenge mercury to a small extent.

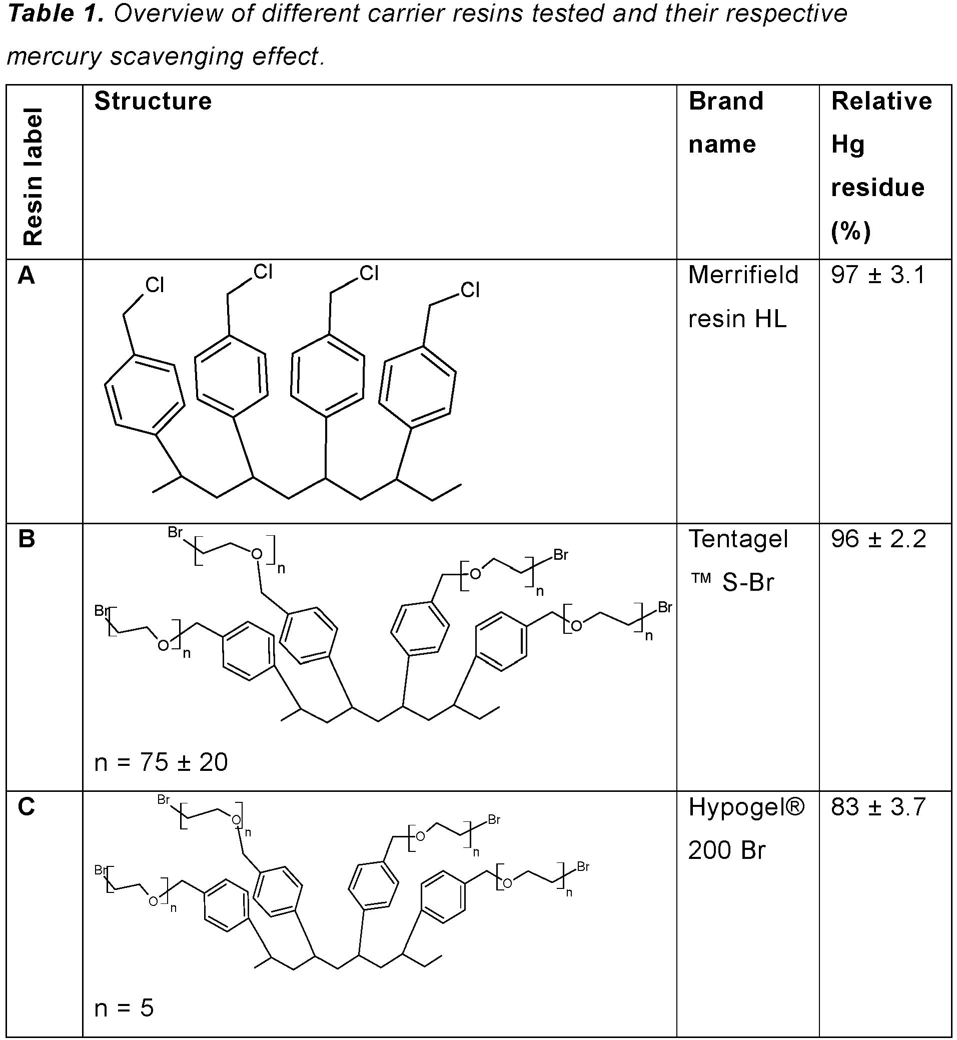

Merrifield Resin is a cross-linked polystyrene resin that carries a chloromethyl functional group.

TentaGel^{®} and HypoGel^{®} are grafted copolymers consisting of a low crosslinked polystyrene matrix on which poly (ethylene glycol) (PEG) is grafted.

TentaGel^{®} is a low cross-linked polystyrene comprising 3,000 - 4,000 MW polyethylene glycol which have been terminally functionalized with a bromide.

HypoGel^{®} is a low cross-linked polystyrene and a very short polyethylene glycol, i.e. pentaethyleneglycol (Mw 220) which has been terminally functionalized as a bromide. HypoGel^{®} is also referred to as O-(2-Bromoethyl)pentaethylene glycol resin.

In Table 2 are shown the mercury binding effect of a series of compounds according to Formula I, in which m+n+o+p is 10, specifically m=3, n=2, o=2 and p=3, where the ring structure is kept constant and the resin is varied. For Scavenger 1, the mercury scavenging ability is lowest whereas for Scavengers 2, and 3, where the polymer is hydrophilic the results are better. This shows the advantage of using a carrier with hydrophilic properties. Notably, resin B, comprising about 5 PEG-groups, is particularly effective.

**Table 2. Scavenger effect of thiacrownether 2-hydroxymethyl-1,4,8, 11, 14-pentathiacycloheptadecane coupled to different resins.**

| **Scavenger number** | **Thiacrownether bound to resin (R** = **resin)** | **Resin** | **Relative Hg residue (%)** |
|---|---|---|---|
| **1** | 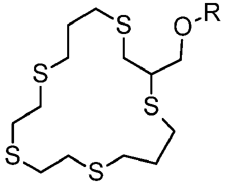 | A | 89 ± 1.6 |
| **2** | 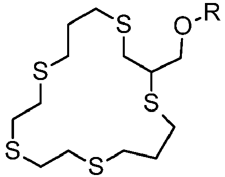 | B | 80 ± 1.9 |
| **3** | 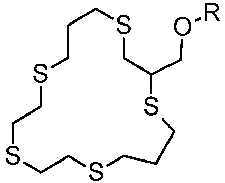 | C | 43 ± 3.2 |

In Table 3, are shown the mercury binding effect of a series of compounds according to Formula I, where the ring structure is varied and the resin is kept constant, to resin C (sold under trade name Hypogel Br). All tested scavengers 3, 4, 5 and 6, in which m+n+o+p is 10 or 8, are able to scavenge more than 50% of the mercury. Notably, Scavengers 4 and 6, in which m+n+o+p=10, were particularly effective. Scavenger 4 (Formula I, where m, n, o, p, q = 2, X is -S-, and A is -CH(CH₂O∼)-) is the most effective. In Scavengers 3 and 4 the linker is a covalent bond and in scavengers 5 and 6 the linker is a methylene group.

**Table 3. Scavenger effect of different thiacrownethers coupled to resin C.**

| **Scavenger number** | **Thiacrownether bound to resin (R= resin)** | **Resin** | **Relative Hg residue (%)** |
|---|---|---|---|
| **3** | 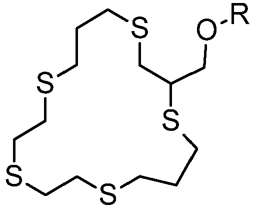 | C | 43 ± 3.2 |
| **4** | 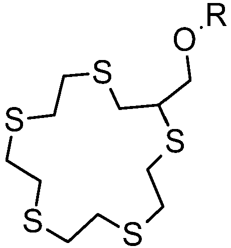 | C | 23 ± 4.0 |
| **5** | 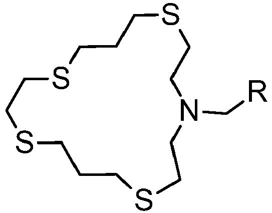 | C | 42 ± 1.8 |
| **6** | 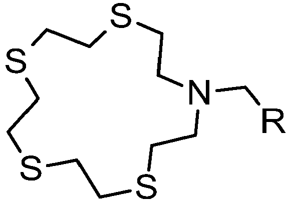 | C | 33 ± 3.7 |

In the Tables 1-3, it can be seen that the resins without the chelating thiacrownethers have some intrinsic effect as mercury scavengers, but for each resin, the mercury scavenging efficiency increases when a compound according to Formula I, is attached. This shows the advantage of the materials of this disclosure as mercury scavengers.

### Preparation of ring structures - Specific Examples

General: Scavenger numbering is the same as in Tables 1, 2, and 3. Materials, reagents and solvents were obtained from commercial sources and were used without further purification unless otherwise noted. NMR spectra (CDCl₃) were recorded on a Varian Unity INOVA400 MHz and chemical shifts are reported relative to the residual solvent peak of the deuterated solvent. IR spectrometer: Thermo Fisher Nicolet iS5. Sulphur content was determined by ICP-OES (Agilent 710).
Abbreviations used: eq - equivalent(s); rt - room temperature.

### Example 1: Scavenger 1.

### Example 1a: 4,7,10-trithiadecane-1,13-diol.

Sodium metal (650 mg) was dissolved in dry ethanol (40 ml) under nitrogen and 3-thiapentane-1,5-dithiol was added and the mixture was heated to reflux until the thiol had dissolved completely. Over a period of 15 min was added 3-bromopropanol and the mixture was refluxed for two h. After the reaction, the solids were removed by filtration and the solvent was evaporated to yield 2.5 g (80%) of TLC (heptane: EtOAc 1:2) pure 4,7,10-trithiadecane-1,13-diol.

### Example 1b: 1,13-dimesylyoxy-4,7,10-trithiatridecane.

To a solution of 4,7,10-trithiadecane-1,13-diol (1 g, 4.1 mmol) in cold (0 °C) DCM (20 ml) and added triethyl amine (1.5 ml, 10.3 mmol) was, over 10 minutes, added methanesulphonyl chloride (0.8 ml, 10.3 mmol). The reaction mixture was allowed to attain room temperature overnight. Aqueous extractive workup with dichloromethane gave a material that was used immediately for the next step.

### Example 1c: 2-hydroxymethyl-1,4,8,11,14-pentathiacycloheptadecane.

To a 3 necked flask was added DBU (4.5 ml, 29 mmol) in dry DMF (100 ml). In another flask the dimesylate from example 1b (5 g, 11.7 mmol) and dimercaptopropanol (1.2 ml, 11.7 mmol) were dissolved in DMF (20 ml) which was then transferred to a 20 ml syringe and was added to the DBU solution by a syringe pump over 40 h. DMF was evaporated and the reaction mixture purified by flash chromatography. It yielded 13% of the product.

### Example 1d: Coupling of 2-hydroxymethyl-1,4,8,11,14-pentathiacyclo-heptadecane to chloromethylated polystyrene (Merrifield's resin).

The resin 200 mg (1 eq) was swelled in DMF. In another reaction flask was taken 54 mg (1.1 eq) of 2-hydroxymethyl-1,4,8,11,14-pentathiacycloheptadecane of example 1c and 10 mg (0.9 eq) of NaH in DMF (10 ml) at 0 °C. It was stirred at that temperature for 30 min and then it was transferred to the resin and was left at rt for 24 hrs. The DMF was filtered off and the resin was successively washed with diethyl ether, DCM and finally with water and dried under vacuum to yield Scavenger 1. The product was characterized by IR.

### Example 2: Scavenger 2

### Coupling of 2-hydroxymethyl-1,4,8,11,14-pentathiacycloheptadecane to bromo-PEG₇₀-polystyrene (Tentagel) to yield scavenger 2.

The resin 500 mg (1 eq) was swelled in DMF. In another reaction flask was taken 50 mg (1.1 eq) of 2-hydroxymethyl-1,4,8,11,14-pentathiacycloheptadecane and 4 mg (0.9 eq) of NaH in DMF (10 ml) at 0 °C. It was stirred at that temperature for 30 min and then it was transferred to the resin and was left at rt for 24 hrs. The DMF was filtered off and the resin was successively washed with diethyl ether, DCM and finally with water and dried under vacuum to yield Scavenger 2. For sulfur content ICP was performed and it was found to be 0.9% w/w. In IR spectra the peak at 686 cm⁻¹ corresponding to the C-Br stretching was missing and also the new peak at 1196 cm⁻¹ indicates the formation of O-C bond.

### Example 3: Scavenger 3

### Coupling of 2-hydroxymethyl-1,4,8,11,14-pentathiacycloheptadecane to bromo-PEGs-polystyrene (Hypogel) to yield Scavenger 3.

The resin 200 mg (1 eq) was swelled in DMF. In another reaction flask was taken 72 mg (1.1 eq) of 2-hydroxymethyl-1,4,8,11,14-pentathiacycloheptadecane and 10 mg (0.9 eq) of NaH in DMF (10 ml) at 0 °C. It was stirred at that temperature for 30 min and then it was transferred to the resin and was left at rt for 24 hrs. The DMF was filtered off and the resin was successively washed with diethyl ether, DCM and finally with water and dried under vacuum to yield Scavenger **3.** Sulfur content from ICP: 0.8% w/w. IR Spectrum: The hydroxyl peak at 3420 cm⁻¹ from the thiocrown ether starting material is missing and a new peak at 1147 cm⁻¹ appeared which indicates an O-C bond.

### Example 4: Scavenger 4:

### Example 4a: 3,6,9-trithiaundecane-1,11-diol.

To a refluxing solution of 3-thiapentane-1,5-dithiol (1 eq) and sodium metal (2 eq) in EtOH was added ethanolic solution of 2-bromoethanol (2 eq) and the resulting mixture was refluxed for 30 min. It was then cooled and filtered through a sintered funnel *in vacuo.* The ethanol was evaporated and the crude was purified by flash chromatography to yield 3,6,9-trithiaundecane-1,11-diol in a yield of 80%

### Example 4b: 1,11-dimesylyoxy-3,6,9-trithiaundecane

To a solution of 3,6,9-trithiaundecane-1,11-diol (2 g, 8.25 mmol) from example 4a in DCM was added triethyl amine (5 ml, 41 mmol) and then added MsCl (9 ml, 83 mmol) at 0 °C and the mixture was stirred at temperature for 30 min and then left at rt overnight. TLC confirmed the consumption of the starting material. Hydrochloric acid (1 M, 10 ml) was added and the organic phase was washed successively washed with water and brine. After drying and evaporation the product was immediately used in the next step without further purification.

### Example 4c: 2-hydroxymethyl-1,4,7,10,13-pentathiacyclopentadecane.

To a 3 necked flask was added DBU (2eq) in dry DMF (100 ml). In another flask the dimesylate from example 4b (1eq) and 1,2-dimercaptopropan-3-ol (1eq) were dissolved in DMF (20 ml) and this solution was then transferred to a 20 ml syringe and was added to the DBU solution by a syringe pump over 48 hours. The reaction bath temperature was set at 60 ⁰C. After 2 days TLC was checked and there was indication of formation of new compound. DMF was evaporated and the reaction mixture purified by flash chromatography. It yielded 19% of the product.

### Example 4d: Coupling of 2-hydroxymethyl-1,4,7,10,13-pentathiacyclo-pentadecane to bromo-PEGs-polystyrene (Hypogel) to yield Scavenger 4.

Hypogel (0.8 mmol/g) was suspended in DMF. In a different flask 2-hydroxymethyl-1,4,7,10,13-pentathiacyclopentadecane (1 eq) was dissolved in DMF and to that was added NaH and it was stirred at 0 ⁰C until it formed a clear solution. Then this was transferred to the suspension of Hypogel and was left stirring for 2 days. After 2 days, the solids were collected on a sintered glass funnel and dried in *vacuo.* Sulfur content: 1.9% (w/w). Final amount 10.5 g.

### Example 5: Scavenger 5.

### Example 5a: 4,7-dithiadecane-1,10-diol.

To a refluxing solution of dithioethylene glycol (1 eq) and sodium metal (2 eq) in EtOH was added ethanolic solution of 3-chloropropanol (1 eq) and the resulting mixture was refluxed further for 30 mins. Then it was cooled and filtered through a sintered funnel in *vacuo.* The ethanol was evaporated and the crude product was purified with flash chromatography to yield 94% of 4,7-dithiadecane-1,10-diol.

### Example 5b: 4,7-dithiadecane-1,10-dithiol.

To an ice cooled solution of 4,7-dithiadecane-1,10-diol (5 g, 27.5 mmol) from example 4a (1 eq) in dry DCM (30 ml) was added thionyl chloride (8 ml, 110 mmol) over one h. The reaction was checked by TLC and found to be essentially complete. The volatiles were evaporated and the intermediate 1,10-dichloro-4,7-dithiadecane was used directly. 4 g was dissolved/suspended in water (10 ml) and thiourea (3 g, 37 mmol) was added. Reflux overnight was followed by the addition of aqueous sodium hydroxide (2 g in 20 ml of water) and reflux again for 6 h. The product was extracted with DCM. Drying over sodium sulphate and evaporation was followed by silica chromatography to yield 4,7-dithiadecane-1,10-dithiol (2.9 g, 74 %) as a viscous liquid, which solidified on standing.

### Example 5c: N-BOC-1-aza-3,7,10,14-tetrathiaheptadecane.

DBU (2 ml) was dissolved in dry DMF (50 ml) in a 3 necked round bottom flask. In another flask was dissolved 4,7-dithiadecane-1,10-dithiol from example 6b (1 g, 4.13 mmol) and N-BOC-bis(2-chloroethyl)-amine (1 g, 4.13 mmol) in DMF (20 ml), which was transferred to a 20 ml syringe and the solution was then added to the DBU solution by a syringe pump over 48 hours. The reaction temperature was set at 60 ⁰C. After 2 days TLC was checked and there was indication of formation of new compound. DMF was evaporated and the residue re-dissolved in DCM and washed with water, 1M HCl and brine solution. The organic phase was dried over Na₂SO₄ and evaporated. It was used in the next step without further purification.

### Example 5d: 1-Aza-3,7,10,14-tetrathiaheptadecane.

The N-BOC-1-aza-3,7,10,14-tetrathiaheptadecane from example 5c was dissolved in in 50% TFA in DCM (5 mL TFA+ 5mL DCM /g) was stirred at rt for 1 h and the solvents were evaporated and the product purified by flash chromatography to get **a** 14% yield of 1-Aza-3,7,10,14-tetrathiaheptadecane based on 4,7-dithiadecane-1,10-dithiol from example 5b.

### Example 5e: Coupling of 1-Aza-3,7,10,14-tetrathiaheptadecane to bromo-PEGs-polystyrene (Hypogel) to yield Scavenger 5.

Hypogel^{®} 200 Br (7 g, 0.8 mmol/g) was suspended in DMF (30 ml). In a different flask 1-aza-3,7,10,14-tetrathiaheptadecane of example 5d (1 g) was dissolved in DMF and to that was added NaH (130 mg) and the mixture was stirred at 0 °C until it formed a clear solution. This was then transferred to the suspension of Hypogel and the mixture was left stirring for 2 days. The derivatized resin was collected on sintered glass funnel and dried in *vacuo.* Sulfur content: 1.02%. Total amount obtained 8.4 gm and to that 1.5 gm of talc was added.

### Example 6: Scavenger 6.

### Example 6a: 1,8-dichloro-3,6-dithiaoctane.

To a solution of 3,6-dithiaoctane-1,8-diol (5 g, 27.5 mmol) in dry DCM (10 ml) was added SOCl₂ (8 ml, 110 mmol) at 0 °C and the mixture was stirred for 1 h and after which the volatiles were evaporated to leave an essentially TLC pure (EtOAc:Heptane 3:1) product which was immediately used for the next step.

### Example 6b: 3,6-dithiaoctane-1,8-dithiol.

To a suspension of the crude 1,8-dichloro-3,6-dithiaoctane (4 g) from example 5a in water (50 ml) was added thiourea (3 g, 37 mmol) and the mixture was refluxed overnight. Aqueous sodium hydroxide (2 g in 30 ml of water) was added and the mixture was refluxed for another 6 h. The product was extracted into dichloromethane. Drying and evaporation yielded the desired product (2.9 g, 74%) as a viscous oil, which solidified on standing, in a TLC pure state.

### Example 6c: N-BOC-1-aza-4,7,10,13-tetrathiacyclopentadecane.

DBU (1.8 ml, 11.7 mmol) was dissolved in dry DMF (50 ml) in a 3 necked round bottom flask. In another flask was dissolved 3,6-dithiaoctane-1,8-dithiol from example 6b (1 g, 4.66 mmol) and N-BOC-bis(2-chloroethyl)-amine (1.12 g, 4.66 mmol) in DMF (20 ml), which was transferred to a 20 ml syringe and the solution was then added to the DBU solution by a syringe pump over 48 hours. The reaction temperature was set at 60 ⁰C. After 2 days TLC was checked and there was indication of formation of new compound. DMF was evaporated and the residue re-dissolved in DCM and washed with water, 1M HCl and brine solution. The organic phase was dried over Na₂SO₄ and evaporated. It was used in the next step without further purification.

### Example 6d: 1-aza-4,7,10,13-tetrathiacyclopentadecane.

The N-BOC-1-aza-4,7,10,13-tetrathiacyclopentadecane from example 6c was dissolved in in 50% TFA in DCM (5 mL TFA+ 5mL DCM /g) was stirred at rt for 1 h and the solvents were evaporated and the product purified by flash chromatography to get **a** 14% yield of 1-Aza-3,7,10,14-tetrathiaheptadecane based on 4,7-dithiadecane-1,10-dithiol from example 5b.

### Example 6e: Coupling of 1-aza-4,7,10,13-tetrathiacyclopentadecane to bromo-PEGs-polystyrene (Hypogel) to yield Scavenger 6.

Hypogel^{®} 200 Br (7 g, 0.8 mmol/g) was suspended in DMF (30 ml). In a different flask 1-aza-4,7,10,13-tetrathiacyclopentadecane of example 6d (1 g) was dissolved in DMF and to that was added NaH (130 mg) and the mixture was stirred at 0 ⁰C until it formed a clear solution. This was then transferred to the suspension of Hypogel and the mixture was left stirring for 2 days. The derivatized resin was collected on a sintered glass funnel and dried in *vacuo.* Sulfur content: 1.02%. Total amount obtained 8.4 g.

## Claims

1. A scavenger comprising a compound according to Formula I coupled to a carrier resin, 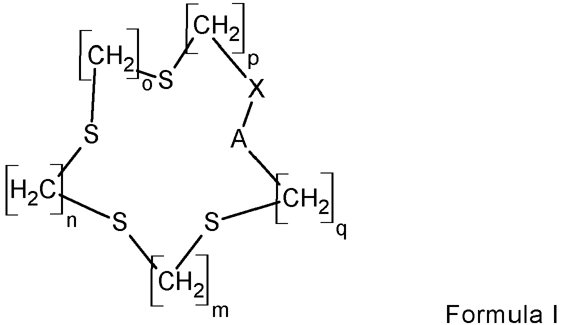 wherein
m, n, o and p independently from each other are 2 or 3;
q is 1 or 2;
X is -S- or >N(~); and
A is >CH(CH₂O∼) when X is -S-, and A is -CH₂- when X is >N(~);
wherein ∼ represents a bond, optionally via a linker, to the carrier resin; and > denotes bonds within Formula I; and
wherein the carrier resin is a polymer comprising hydrophilic groups.

2. The scavenger according to claim 1, wherein the hydrophilic groups have a molecular composition of (a+b)/c ≥ 0.25 where a, b, and c are the mol percentage of oxygen (O), the mol percentage of nitrogen (N), and the mol percentage of the sum of all non-hydrogen atoms, respectively.

3. The scavenger according to claim 1 or 2, where the hydrophilic groups are polyethylene glycol, preferably wherein the polyethylene glycol has a polymerization degree of 5-100.

4. The scavenger according to claim 2 or 3, wherein the carrier resin comprises polystyrene.

5. The scavenger according to any one of the preceding claims, wherein m+n+o+p=10 or wherein m+n+o+p=8.

6. The scavenger according to any one of the preceding claims, wherein X is -S-.

7. The scavenger according to any one of claims 1 to 6, wherein m, n, o and p are 2; and q is 1.

8. The scavenger according to any one claims 1 to 5, wherein
m and p are 3; n and o are 2; q is 1; and X is -S-; or
m and o are 3; n and p are 2; q is 1; and X is >N(~).

9. A scavenger for use as a medicament, wherein the scavenger comprises a compound according to Formula I coupled to a carrier resin, 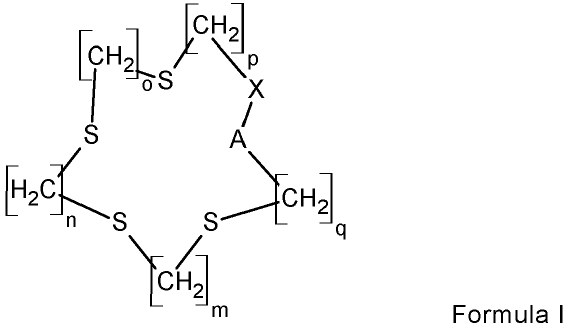 wherein
m, n, o and p independently from each other are 2 or 3;
q is 1 or 2;
X is -S- or >N(∼); and
A is >CH(CH₂O∼) when X is -S-, and A is -CH₂- when X is >N(∼);
wherein ~ represents a bond, optionally via a linker, to the carrier resin; and > denotes bonds within Formula I; and
wherein the carrier resin is a resin having a total molecular composition of (a+b)/(c) < 0.32, where a, b, and c are the mol percentage of oxygen (O), the mol percentage of nitrogen (N), and the mol percentage of the sum of all non-hydrogen atoms, respectively.

10. The scavenger for use according claim 9, wherein the carrier resin is chosen from the group consisting of polystyrene or PEG-polystyrene, preferably wherein the carrier resin is PEG-polystyrene.

11. The scavenger for use according claim 9 or 10, wherein m+n+o+p=10 or wherein m+n+o+p=8.

12. The scavenger for use according to any one of claims 9 to 11, wherein X is -S-.

13. The scavenger for use according to any one of claims 9 to 12, wherein m, n, o and p are 2; q is 1.

14. The scavenger for use according to any one of claims 9 to 11, wherein
m and p are 3; n and o are 2; q is 1; and X is -S-; or
m and o are 3; n and p are 2; q is 1; and X is >N(~).

15. The scavenger for use according to any one of claims 9 to 14, wherein the scavenger is comprised in a formulation chosen from the group consisting of a capsule, a tablet, a powder, a suspension, and an effervescent tablet.

16. The scavenger for use according to any one of claims 9 to 15 for use in the treatment and/or alleviation and/or prevention of diseases and/or conditions chosen from the group consisting of autoimmune diseases, neurological diseases, fibromyalgia, chronic fatigue syndrome, chronic obstructive pulmonary disease, chronic bronchitis, and heavy metal poisoning.

17. The scavenger for use according to claim 16 for use in the treatment and/or alleviation and/or prevention of heavy metal poisoning, wherein the heavy metal is mercury.

18. Method of preparation of a compound according to Formula I, 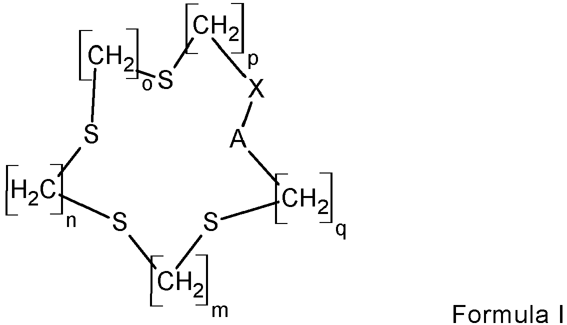 wherein
m, n, o and p independently from each other are 2 or 3;
q is 1 or 2;
X is -S-; and
A is >CH(CH₂O∼);
wherein ~ represents H; and > denotes bonds within Formula I;
wherein the method comprises the steps of:
(a) providing a solution of 1,2-dimercaptopropan-3-ol and a compound according to Formula II,
MsO-(CH₂)ᵣ-S-(CH₂)ₛ-S-(CH₂)ₜ-S-(CH₂)ᵤ-OMs Formula II
wherein r, s, t, and u independently of each other are 2 or 3, in a polar aprotic solvent;
(b) providing a solution of a non-nucleophilic base in a polar aprotic solvent;
(c) adding the solution of step (a) to the solution of step (b) over a time period of 24 h or longer, while heating the mixture to a temperature of 50-150 °C;
(d) removing the solvents; and
(e) purifying the compound according to Formula I.
